# EUROPÄISCHE PATENTSCHRIFT

(11) **EP 4 233 832 B1**
(45) Veröffentlichungstag und Bekanntmachung des Hinweises auf die Patenterteilung: **01.04.2026**
(21) Anmeldenummer: 22158419.6
(22) Anmeldetag: 24.02.2022
(51) Int. Cl.: A61K 8/34, A61K 8/49, A61Q 17/04, A61K 8/04

(54) **TRANSPARENTES SONNENSCHUTZMITTEL MIT HOHEM UVA SCHUTZ**
TRANSPARENT SUNSCREEN COMPOSITION WITH HIGH UVA PROTECTION
MILIEU TRANSPARENT DE PROTECTION SOLAIRE À HAUTE PROTECTION CONTRE LES UVA

(43) Veröffentlichungstag der Anmeldung: 30.08.2023
(73) Patentinhaber: Dermcos GmbH, 69190 Walldorf (DE)
(72) Erfinder: Müller, Stefan, 79576 Weil am Rhein (DE); Kozlova, Diana, 69231 Rauenberg (DE)
(74) Vertreter: Krauss, Jan

(56) Entgegenhaltungen:
- WO-A1-96/41613
- DE-A1- 102005 059 742
- DE-U1- 202010 006 005
- US-A1- 2016 158 132
- ANDERSEN ALAN F: "Final Report of the Safety Assessment of Alcohol Denat., Including SD Alcohol 3-A, SD Alcohol 30, SD Alcohol 39, SD Alcohol 39-B, SD Alcohol 39-C, SD Alcohol 40, SD Alcohol 40-B, and SD Alcohol 40-C, and the Denaturants, Quassin, Brucine Sulfate/Brucine, and Denatonium Benzoate1", vol. 27, no. 1_suppl, 1 January 2008 (2008-01-01), US, pages 1 - 43, XP055955237, ISSN: 1091-5818, Retrieved from the Internet <URL:http://journals.sagepub.com/doi/pdf/10.1080/10915810802032388> [retrieved on 20220825], DOI: 10.1080/10915810802032388
- BURNETT MARK E. ET AL: "Sunscreens: Obtaining adequate photoprotection", DERMATOLOGIC THERAPY, vol. 25, no. 3, 1 May 2012 (2012-05-01), US, pages 244 - 251, XP055955043, ISSN: 1396-0296, DOI: 10.1111/j.1529-8019.2012.01503.x
- PATIL ANJALI ET AL: "Chemistry, Process Design, and Safety for the Nitration Industry /ACS /Symposium Series", vol. 1148, 1 January 2013 (2013-01-01), US, pages 13 - 37, XP055954998, ISSN: 0097-6156, Retrieved from the Internet <URL:https://pubs.acs.org/doi/pdf/10.1021/bk-2013-1148.ch002> DOI: 10.1021/bk-2013-1148.ch002

## Beschreibung

Die vorliegende Erfindung betrifft ein alkoholbasiertes kosmetisches transparentes Sonnenschutzspray ohne filmbildende Polymere und Octocrylene, jedoch mit dem wasserlöslichen Lichtschutzfilter Phenylbenzimidazolsulfonat unter Ausschluss des Vergällungsmittels Methylethylketon, insbesondere in Form eines Pumpzerstäubersprays oder eines Aerosol-Sprays.

### Hintergrund der Erfindung

Die vorliegende Erfindung betrifft eine kosmetische, transparente Zubereitung mit Lichtschutzfilter als Sonnenschutzmittel für die menschliche Haut.

Seit Jahrzehnten setzen sich die Menschen in vielen Teilen der Erde der Sonnenstrahlung aus, da diese eine Pigmentbildung im Sinne einer Melaninbildung hervorruft und die Haut gebräunt wird. Die ultraviolette Strahlung der Sonne hat jedoch auch eine schädigende Wirkung auf die Haut. Neben der akuten Schädigung, dem Sonnenbrand treten auch Langzeitschäden wie ein erhöhtes Hautkrebsrisiko oder vorzeitige Hautalterung auf.

Zum Schutz der Haut wurden daher eine Reihe von Lichtschutzfiltersubstanzen entwickelt, die in kosmetischen Zubereitungen eingesetzt werden können. Die verwendbaren UVA-, UVB-, oder Breitbandlichtschutzfilter sind insbesondere in der europäischen Kosmetikverordnung KVO 1223/2009 gelistet.

Diese Lichtschutzfilter werden in einer Vielzahl unterschiedlicher kosmetischer Darreichungsformen kommerziell angeboten, um dem Anwender einen Sonnenschutz für die unterschiedlichsten Einsatzsituationen (beim Sport, beim Sonnenbad am Stand und bei sonstigen Freizeitaktivitäten) und für unterschiedlichen Körperpartien (Gesicht, Augenbereich, Lippen und Ganzkörperbereich) anbieten zu können. Daraus entwickelten sich Anwendungen als Cremes, Fluide, Lotionen, Milch, Aerosolschäume, Emulsionsspray - sogar Tücher und Lippenstifte mit Sonnenschutz befinden sich im kommerziellen Angebot.

In den letzten Jahren erfreuen sich insbesondere Sonnensprays zunehmender Beliebtheit bei den Konsumenten, vor allem wegen der einfachen Anwendung. In der Freizeit und Sportanwendung hat sich eine noch speziellere Anwendung durchgesetzt; das transparente alkoholische Sonnenspray. Diese neue Anwendungsform stellt eine Lösung und keine Emulsion dar, ist daher besonders gut auf der Haut verteilbar und hinterlässt einen wenig klebrigen Film. Die von Anwendern oft kritisierte Klebrigkeit von Sonnenschutzprodukten fällt bei dieser Anwendungsform daher besonders gering aus.

Jedoch weisen diese transparenten alkoholischen Sonnensprays oder -gele auch einen Nachteil auf. Durch den Verzicht auf UV-absorbierende Pigmente wie Titandioxid ist man in der Auswahl der UV-Filter eingeschränkt. Es können daher nur lösliche UV-Filter eingesetzt werden. Im Rahmen der behördlich zugelassenen Einsatzkonzentrationen dieser UV-Filter sind hohe Lichtschutzfaktoren bei gleichzeitig hohem bis sehr hohem UVA-Schutz nur schwer realisierbar.

Transparente Sonnenschutzsprays, die einen hohen Lichtschutzfaktor und gleichzeitig einen hohen UVA Schutz aufweisen sollen, bedürfen nach dem derzeitigen Stand der Technik einerseits einen sehr hohen Einsatz an öllöslichen UV-Filtern. Andererseits bedürfen Sie der Zugabe eines filmbildenden Polymers, das zur Bildung eines homogenen Schutzfilms für hohe Lichtschutzfaktoren als unverzichtbar gilt. Auch der filmbildende aber klebrige UV-Filter Octocrylene wird gemäß dem Stand der Technik sehr häufig eingesetzt, um die Bildung eines gleichmäßigen, aber klebrigen Films auf der Haut zu unterstützen. Gleichzeitig unterstützt das Octocrylene die notwendige Photostabilität der UVA-Filter, insbesondere die des Avobenzons.

In der WO 2007/068699 A1 ist der bisherige Aufbau von transparenten alkoholischen Sprays und Gelen detailliert beschrieben. Diese werden als alkoholische Lösungen mit organischen öllöslichen UV-Filtern und filmbildenden Polymeren, wie zum Beispiel Acrylat/Octylacrylamid-Copolymer, Carbomer oder Acrylat/C10-30 Alkyl-Acrylat Crosspolymer, auch anhand zahlreicher Beispiele vorgestellt.

In den letzten Jahren sind jedoch der UV-Filter Octocrylene und die filmbildenden synthetischen, oft Acrylat-basierten Polymere (synthetisches Polymer) in öffentliche Kritik geraten. Das Octocrylene einerseits, weil Untersuchungen nahelegen, dass bei Lagerung potentiell krebserregende Benzophenon-Derivate entstehen, und das synthetische Polymer andererseits, weil es als potentiell gewässerschädigend angesehen wird.

Leichte, gut einziehende und nicht klebrige alkoholisch-transparente Sprays mit einem hohen Lichtschutzfaktor bei gleichzeitig hohen UVA-Schutz sind insbesondere für Menschen mit heller Haut oder für ältere Menschen mit aktinischer Keratose von besonderem Interesse, da diese Anwendergruppe bislang nicht auf diesen vorteilhaften Produkttyp transparenter alkoholischer Sprays ohne eine klebrige Filmbildung zugreifen kann.

Aufgabe der vorliegenden Erfindung ist es daher, diesen Mangel des Standes der Technik zu beseitigen und eine alkoholisch-transparente kosmetische Zubereitung zu schaffen, welche einen hohen Lichtschutzfaktor mit gleichzeitig hohem UVA-Schutz ermöglicht, ohne die Nutzung der Filmbildung, also z.B. ohne die unerwünschten Zutaten Octocrylene und ohne synthetisches Polymer.

Diese Aufgabe wird erfindungsgemäß gelöst, indem eine transparente kosmetische Sonnenschutz-Zusammensetzung auf alkoholischer Basis zur Verfügung gestellt wird, umfassend von 0,1 bis 3 %, bezogen auf die gesamte Zubereitung, des UV-Filters Phenylbenzimidazolsulfonat, mindestens ein kosmetisch akzeptables Öl, gelöst in Ethylalkohol, mit einem Wasseranteil von 0.1 bis zu 10 %, bezogen auf die gesamte Zubereitung und wobei die Zusammensetzung frei von Octocrylene und filmbildendem synthetischem Polymer und frei von dem Vergällungsmittel Methylethyl-Keton (MEK). Bevorzugt wird weiter das Phenylbenzimidazolsulfonat durch wässriges Neutralisieren der Phenylbenzimidazolsulfonsäure in Wasser erzeugt.

Die Gebrauchsmusterschrift DE 20 2010 006 005.2 befindet sich im vorliegenden Gebiet, grenzt sich jedoch von der vorliegenden Erfindung durch eine filmbildende Anwendung mit Polymeren und Octocrylene ab. Darüber hinaus kann bei dieser Beschreibung durch den Einsatz von MEK-denaturiertem Alkohol ein unangenehmer Geruch entstehen, der nur durch eine sehr geringe Dosierung von Phenylbenzimidazolsulfonat und einer starken Parfümierung entgegengewirkt und bekämpft werden kann.

Überraschend und unerwartet war für den Fachmann, dass sich durch die vorliegende Erfindung ohne jede polymerbasierte Filmbildung dennoch ein hoher Lichtschutzfaktor bei gleichzeitig hohem und hinreichend photostabilem UVA-Schutz realisieren lässt. Darüber hinaus konnte durch Verwendung eines unvergällten oder lediglich durch Isopropylalkohol vergällten Ethanols die Entstehung des unangenehmen Geruchs auch bei höherer Dosierung von Phenylbenzimidazolsulfonat (0,5-3 %) überraschend abgewendet wird.

Die erfindungsgemäße Zusammensetzung ist transparent. Dabei gilt eine Zusammensetzung erfindungsgemäß als transparent, wenn es möglich ist, bei Tageslicht mit dem bloßen Auge durch eine mit der erfindungsgemäßen Zusammensetzung gefüllten Einmal-Küvette (Firma Brand, 2,5 ml, Wellenlängenbereich: 220 nm-900 nm) hindurch zu schauen. Schriftzeichen (Schrifttyp Anal Schriftgröße 8), die sich unmittelbar hinter der Einmal-Küvette befinden, müssen klar erkennbar und lesbar sein. Erfindungsgemäß bevorzugt enthält die erfindungsgemäße Zusammensetzung von 30 bis 85 Gew.-% Ethanol und ganz besonders bevorzugt 50-80 Gew.-% Ethanol, jeweils bezogen auf das Gesamtgewicht der Zubereitung. Der Wasseranteil der erfindungsgemäßen Zusammensetzung liegt bevorzugt unter 10%, besonders bevorzugt unterhalb 3% aber mindestens 0,1 %. Zusätzlich kann die Zusammensetzung 1 bis 50 % Isopropylalkohol enthalten.

In bevorzugter Ausgestaltung der Erfindung enthält die Zusammensetzung von 10 bis 45 Gew.-% von bei 20°C flüssigen Ölkomponenten und ganz besonders bevorzugt 15 bis 45 Gew.-% bei 20°C flüssigen Ölkomponenten, jeweils bezogen auf das Gesamtgewicht der Zusammensetzung.

In weiterer Ausgestaltung der Erfindung können die bei 20°C flüssigen Ölkomponenten ausgewählt werden aus der Gruppe der Verbindungen C12-15-Alkylbenzoat, Butylenglykol Dicaprylat/Dicaprat, Octyldodekanol, Phenethylbenzoat, Cocoglycerid, Isopropylpalmitat, Ethylhexylstearat, Dicaprylylether, Dicaprylylcarbonate, Undecane, Tridecane, Dimeticone, Alkyl Dimeticone, Caprylic/Capric Triglyceride.

Es ist bevorzugt im Sinne der vorliegenden Erfindung, wenn die flüssigen Pflegekomponenten Glycerin mit 2-10%, und Vitamin E Derivate, wie Tocopherylacetat mit 0,2 bis 2% und/oder Panthenol mit 0,2 bis 2% als weitere Pflegekomponenten in der Zusammensetzung eingesetzt werden.

Bevorzugt umfasst die Zusammensetzung weiter mindestens einen weiteren öllöslichen UV-Filter. Als insbesondere weitere öllösliche UV-Filter werden im Sinne der vorliegenden Erfindung als vorteilhaft eingesetzt: Butylmethoxydibenzoylmethan (Avobenzone), Ethylhexylsalicylat (EHS), Diethylhexyl-Butamidotriazon (DBT), 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) (BEMT), Ethylhexyltriazon (EHT), sowie Diethylamino hydroxybenzoyl hexyl benzoat (DHHB) eingesetzt. Darüber hinaus können auch alle anderen öllöslichen UV-Filter, die im Anhang VI der Kosmetikverordnung KVO 1223/2009 aufgeführt sind, eingesetzt werden. Bevorzugt ist Avobenzone.

Bevorzugt ist eine Zubereitung der Erfindung, die ein UVA/UVB-Verhältnis von mindestens 0,7 oder mehr, bevorzugt von mindestens 0,9 oder mehr aufweist.

Im Unterschied zum herkömmlichen Stand der Technik und dem in der WO 2007/068699 beschriebenen Sonnenschutzmittel ist es im Sinne dieser vorliegenden Erfindung nicht vorteilhaft, die UV-Filter Titandioxid oder Zinkoxid einzusetzen, da dies zu einem Verlust der gewünschten Transparenz des Sonnenschutzmittels führt. Die Zubereitung ist somit bevorzugt frei von Titandioxid und/oder Zinkoxid.

Zum Neutralisieren der 2-Phenylbenzimidazol-5-sulfonsäure (INCI: Phenylbezimidazol sulfonic acid: PBSA) kann im Sinne der Erfindung vorteilhaft das Aminomethylpropanol, eine wässrige Natronlauge oder eine wässrige Kalilauge eingesetzt werden. Auch andere Amine, wie das Tetrahydroxypropyl-Ethylendiamin, können hierbei zum Einsatz kommen.

Ein weiterer Aspekt der vorliegenden Erfindung betrifft daher ein Verfahren zu Herstellung einer erfindungsgemäßen kosmetische Zusammensetzung, umfassend ein geeignetes Formulieren der Inhaltsstoffe der kosmetischen Zusammensetzung wie hier beschrieben, weiter umfassend den Schritt des Herstellens des Phenylbenzimidazolsulfonats durch wässrige oder alkoholische Neutralisation der Phenylbenzimidazolsulfonsäure vor der Zusammenstellung/Formulierung.

Die vorteilhafte Einsatzkonzentration des PBSA liegt erfindungsgemäß zwischen 0,1 Gew.-% und 5 Gew.-%, besonders vorteilhaft zwischen 0,3 Gew.-% und 3 Gew.-% und ganz besonders vorteilhaft zwischen 0,3 Gew.-% und 1,5 Gew.-% bezogen auf die kosmetische Zubereitung.

Als weitere erfindungsgemäße Einsatzstoffe kommen Organosiloxane, wie Cyclomethicon oder Dimethicon zum Einsatz, Pflegestoffe wie Vitamin E, Vitamin C, Vitamin A oder deren Derivate, sowie andere Hilfsstoffe wie Propylenglycol, Isopropylalkohol oder flüssige Emulgatoren in Betracht. Auch weitere Einsatzstoffe wie Ronacare AP oder Ubiquinone, Flavonoide oder Antifalten-Wirkstoffe wie Tripeptide oder Ectoin sind nicht limitierend im Sinne der vorliegenden Erfindung.

Es ist erfindungsgemäß vorteilhaft, wenn die kosmetische Darreichungsform ein alkoholisches Spray darstellt. Es ist erfindungsgemäß weiter vorteilhaft, wenn die erfindungsgemäße Zubereitung als Spray mit Pumpzerstäuber oder als Aerosol mit einem oder mehreren Treibgasen gewählt aus der Gruppe, Propan, Butan, Isobutan, Pentan/Isopentan oder Dimethylether (DME), anzuwenden. Ferner ist erfindungsgemäß vorteilhaft, das Spray in Form eines Bag-on-Valve-Aerosols darzustellen. Ferner ist erfindungsgemäß vorteilhaft, wenn die kosmetische Darreichungsform ein Aerosol-Sprays mit einem oder mehreren der Treibgase Propan, Butan, Isobutan, Pentan, Isopentan oder Dimethylether darstellt.

Ein weiterer Aspekt der vorliegenden Erfindung betrifft die erfindungsgemäße Zusammensetzung zur Verwendung zu einer Prävention oder Behandlung von UV-basierten Hautschäden, wie Sonnenbrand oder Hautkrebs sowie aktinischer Keratose oder vorzeitiger Hautalterung, umfassend die topische Applikation einer effektiven Menge der Zusammensetzung auf die Haut eines Säugetiers, insbesondere eines Menschen. Dabei erhöht sich insbesondere die Compliance durch die vorteilhafte Zusammensetzung des Produktes, bei Aufrechterhaltung des Schutzes.

Das nachfolgende erfindungsgemäße Beispiel soll die vorliegende Erfindung verdeutlichen, ohne sie jedoch einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Zu einer weiteren Verdeutlichung des erfindungsgemäßen Vorteils; es handelt sich bevorzugt um ein transparentes Sonnenspray mit dem hohen Lichtschutzfaktor 50 (in-vivo bestimmt: 55) mit einem UVA/UVB-Verhältnis von 0,92, es erfüllt damit die hohen UVA-Schutzkriterien des Boots-Star-Ratings (5 Sterne).

Das UVA/UVB-Verhältnis kann typischerweise wie folgt bestimmt werden. 0,75, 1,0 oder 1,3 mg/cm2 (je nach Methode) der zu testenden Zusammensetzung werden auf ein geeignet angerauhtes Substrat aufgetragen (überprüft durch ein Profilometer). Nach dem kontrollierten Verteilen wird die Probe für mindestens 15 oder 30 Minuten getrocknet. Dann wird die erste UV Absorptionsmessung für die verschiedenen Platten mittels eines Spektrophotometers (UV Analyzer) durchgeführt, um die Sonnenschutzfaktoren für die UV Dosis-Aussetzungsberechnung (falls erforderlich) zu berechnen. Danach werden die Proben gegenüber UV Licht aus einem kalibrierten Solarsimulator ausgesetzt, der eine präzise Dosis (unter Verwendung eines Radiometers überprüft) zuführt (UV Quelle unter Verwendung eines Spektrophotometers überprüft), und dann wird eine zweite UV Absorptionsmessung durchgeführt. Das UVA/UVB-Verhältnis (Boots Star Bewertungssystem) wird dann final aus dem Verhältnis der mittleren UVA Absorption zur mittleren UVB Absorption berechnet, beide definiert durch die Fläche (pro Einheit Wellenlänge) des jeweiligen UV Teils aus derselben Kurve.

Andere Bestimmungen können wie in Pelizzo et al. (In vitro evaluation of sunscreens: an update for the clinicians. *ISRN Dermatol.* 2012;2012:352135. doi:10.5402/2012/352135) erfolgen, z.B. gemäß COLIPA 2011/FDA Final Rule 2011 unter der Verwendung von UV/Vis LAMBDA Spektrophotometern.

### Beispiel:

| | Transp. Sonnenspray LSF 50/ UVA/UVB= 0,92 | | |
|---|---|---|---|
| | | | |

| | Rohstoff | Gew.-% | |
|---|---|---|---|
| | | | |
| 1 | Alkohol Denat. (IPA/TB ohne MEK) | 53,7 | |
| 2 | Glycerin | 3 | |
| 3 | DHHB | 3,5 | |
| 4 | K-Phenylbenzimidazolsulfonat | 0,8 | |
| 5 | Wasser | 0,5 | |
| 6 | Kalilauge, 30 % (KOH) | 0,3 | |
| 7 | BEMT | 2,8 | |
| 8 | Avobenzone | 4,9 | |
| 9 | EHS | 4,9 | |
| 10 | C12-15 Alkyl Benzoat | 18 | |
| 11 | Dicaprylyl Ether | 5 | |
| 12 | Ethylhexyltriazon | 2,2 | |
| 13 | DBT | 0,2 | |
| 14 | Vitamin E Acetat | 0,2 | |
| | | | |
| | | | |
| | | 100 | |
| | | | |

## Patentansprüche

1. Transparente kosmetische Sonnenschutz-Zusammensetzung auf alkoholischer Basis, umfassend
von 0,1 bis 3 Gew.-%,
bezogen auf die gesamte Zubereitung, des UV-Filters Phenylbenzimidazolsulfonat,
mindestens ein kosmetisch akzeptables Öl,
gelöst in Ethylalkohol,
mit einem Wasseranteil von 0,1 % bis zu 10 Gew.-%,
bezogen auf die gesamte Zubereitung,
wobei die Zusammensetzung frei von Octocrylene und filmbildendem synthetischem Polymer und frei von dem Vergällungsmittel Methylethyl-Keton (MEK) ist.

2. Kosmetische Zusammensetzung nach Anspruch 1, weiter umfassend einen weiteren öllöslichen UV-Filter, bevorzugt Avobenzone.

3. Kosmetische Zusammensetzung nach Anspruch 1 oder 2, weiter umfassend Glycerin, Vitamin E Acetat und/oder Panthenol.

4. Kosmetische Zusammensetzung nach einem der voranstehenden Ansprüche, die ein UVA/UVB-Verhältnis von mindestens 0,7 oder mehr, bevorzugt von mindestens 0,9 oder mehr aufweist.

5. Kosmetische Zusammensetzung nach einem der voranstehenden Ansprüche, wobei die kosmetische Darreichungsform ein alkoholisches Spray darstellt.

6. Kosmetische Zusammensetzung nach einem der voranstehenden Ansprüche, wobei die kosmetische Darreichungsform eines Aerosol-Sprays mit einem oder mehreren der Treibgase Propan, Butan, Isobutan, Pentan, Isopentan oder Dimethylether darstellt.

7. Kosmetische Zusammensetzung nach einem der voranstehenden Ansprüche, wobei das Phenylbenzimidazolsulfonat durch wässrige oder alkoholische Neutralisation von Phenylbenzimidazolsulfonsäure hergestellt wird.

8. Verfahren zu Herstellung einer kosmetischen Zusammensetzung nach einem der voranstehenden Ansprüche, umfassend ein geeignetes Formulieren der Inhaltsstoffe der kosmetischen Zusammensetzung wie hier beschrieben, und weiter umfassend den Schritt des Herstellens des Phenylbenzimidazolsulfonats durch wässrige oder alkoholische Neutralisierung der Phenylbenzimidazolsulfonsäure vor der Formulierung.

9. Kosmetische Zusammensetzung nach einem der voranstehenden Ansprüche 1-7 zur Verwendung bei der Prävention oder Behandlung von UV-basierten Hautschäden, wie Sonnenbrand oder Hautkrebs sowie aktinischer Keratose oder vorzeitiger Hautalterung bei einem Säugetier, insbesondere einem Menschen.

## Claims

1. Transparent cosmetic sun protective composition on an alcoholic basis, comprising,
from 0.1 to 3 wt % with respect to the overall composition of the UV filter phenylbenzimidazole sulfonate,
at least one cosmetically acceptable oil,
dissolved in ethyl alcohol,
having a water content of 0.1 % up to 10 wt % with respect to the overall composition,
wherein the composition is free from octocrylene and film forming synthetic polymer and is free from the denaturant methyl ethyl ketone (MEK).

2. Cosmetic composition according to claim 1, further comprising an additional oil-soluble UV-filter, preferred Avobenzone.

3. Cosmetic composition according to claim 1 or 2, further comprising glycerin, vitamin E acetate and/or panthenol.

4. Cosmetic composition according to any one of the preceding claims, having an UVA/UVB-ratio of at least 0.7 or more, preferably of at least 0.9 or more.

5. Cosmetic composition according to any one of the preceding claims, wherein the cosmetic administration form is an alcoholic spray.

6. Cosmetic composition according to any one of the preceding claims, wherein the cosmetic administration is an aerosol spray with one or several of the propellants propane, butane, isobutane, pentane, isopentane or dimethylether.

7. Cosmetic composition according to any one of the preceding claims, wherein the phenylbenzimidazole sulfonate is produced through an aqueous or alcoholic neutralization of phenylbenzimidazole sulfonic acid.

8. Method for producing a cosmetic composition according to any one of the preceding claims, comprising a suitable formulating of the ingredients of the cosmetic composition as described herein, and further comprising the step of producing the phenylbenzimidazole sulfonate through an aqueous or alcoholic neutralization of phenylbenzimidazole sulfonic acid before the formulation.

9. Cosmetic composition according to any one of the preceding claims 1 to 7 for use in the prevention or treatment of UV-based skin damage, such as sunburn or skin cancer as well as actinic keratosis or premature skin ageing in a mammal, in particular a human.

## Revendications

1. Composition cosmétique transparente de protection solaire à base alcoolique, comprenant
de 0,1 à 3 % en poids, par rapport à l'ensemble de la préparation, du filtre UV phénylbenzimidazolsulfonate,
au moins une huile cosmétiquement acceptable,
dissoute dans de l'éthanol,
avec une teneur en eau comprise entre 0,1 % et 10 % en poids, par rapport à l'ensemble de la préparation,
dans laquelle la composition est exempte d'octocrylène, de polymère synthétique filmogène et de l'agent dénaturant méthyléthyl-cétone (MEK).

2. Composition cosmétique selon la revendication 1, comprenant en outre un autre filtre UV liposoluble, de préférence l'avobenzone.

3. Composition cosmétique selon la revendication 1 ou 2, comprenant en outre du glycérol, de l'acétate de vitamine E et/ou du panthénol.

4. Composition cosmétique selon l'une des revendications précédentes, présentant un rapport UVA/UVB d'au moins 0,7 ou plus, de préférence d'au moins 0,9 ou plus.

5. Composition cosmétique selon l'une des revendications précédentes, dans laquelle la forme cosmétique de délivrance est un spray alcoolique.

6. Composition cosmétique selon l'une des revendications précédentes, dans laquelle la forme cosmétique de délivrance est un spray aérosol, contenant un ou plusieurs des gaz propulseurs suivants : propane, butane, isobutane, pentane, isopentane ou diméthyléther.

7. Composition cosmétique selon l'une des revendications précédentes, dans laquelle le phénylbenzimidazolsulfonate est obtenu par neutralisation aqueuse ou alcoolique d'acide phénylbenzimidazole sulfonique.

8. Procédé de fabrication d'une composition cosmétique selon l'une des revendications précédentes, comprenant une formulation appropriée des ingrédients de la composition cosmétique telle que décrite ici, et comprenant en outre l'étape consistant à produire le phénylbenzimidazolsulfonate par neutralisation aqueuse ou alcoolique d'acide phénylbenzimidazole sulfonique avant la formulation.

9. Composition cosmétique selon l'une des revendications 1 à 7, destinée à être utilisée dans la prévention ou le traitement des dommages cutanés induits par les UV, tels que les coups de soleil, le cancer de la peau, la kératose actinique ou le vieillissement prématuré de la peau chez un mammifère, en particulier un être humain.
